# EUROPEAN PATENT APPLICATION

(11) **EP 2 474 287 A1**
(43) Date of publication of application: **11.07.2012**
(21) Application number: 11150640.8
(22) Date of filing: 11.01.2011
(51) Int. Cl.: A61F 2/24, A61F 2/84

(54) **Delivery catheter for stent-valve, and sub-assembly therefor**

(71) Applicant: Symetis SA, 1012 Lausanne (CH)
(72) Inventor: Lombardi, Fabien, 1008 Prilly (CH); Hefti, Jean-Luc, 1400 Cheseaux-Noréaz (CH); Biadillah, Youssef, 1006 Lausanne (CH)
(74) Representative: Hepp Wenger Ryffel AG

(57) **Abstract**

A stent-valve delivery catheter comprises a flexible sub-assembly for transmitting a pushing force from a proximal portion to a distal portion of the catheter. The sub-assembly comprises a tubular support carrying a segmented tube. The segmented tube comprises first segments of a first elastic modulus alternating with second segments of a second elastic modulus. The first segments may be of metal, the second segments of plastics. The sub-assembly combines flexibility provided by the second segments with axial support bearing axial compression force provided by the first segments.

## Description

The present invention relates to a delivery catheter for a stent. In some embodiments, the delivery catheter is intended to be introduced into the human vasculature. In some embodiments, the delivery catheter is intended to deliver a stent-valve. The stent-valve may, for example, be a cardiac stent-valve. The delivery system may be for use as part of a tran-scatheter aortic valve implantation (TAVI) procedure.

The following presents a simplified summary of the invention in order to provide a basic understanding of some aspects of the invention. This summary is not an extensive overview of the invention. It is intended to neither identify key or critical elements of the invention nor delineate the scope of the invention. Its sole purpose is to present some concepts of the invention in a simplified form as a prelude to the more detailed description that is presented later.

In one aspect, the invention provides a delivery catheter for a stent, the delivery catheter including a structure and/or sub-assembly comprising a flexible support tube and a segmented tube nested one within the other. The segmented tube may comprise segments arranged contiguously end to end. The segments may include plural first segments having a first elastic modulus, interposed at least one between plural second segments having a second elastic modulus different from the first elastic modulus.

In some embodiments, the support tube is a flexible core around which the segmented tube is arranged as a sleeve. Alternatively, the segmented tube may be disposed within the lumen of the support tube.

In one form, the segments may be arranged in repeating sequence of a first segment interposed contiguously between second segments adjacent to the first segment. For example, the segments may define a continuous and/or repeating pattern of alternating first and second segments.

The first elastic modulus may be greater than the second elastic modulus, for example, at least 10 times as great, or at least 100 times as great, or at least 500 times as great, or at least 1000 times as great.

The first or the second segments may be of the same material as the core. Alternatively, both the first and second segments may be of different material(s) from the core.

The first and second segments may be of the same material or of different materials. The first and second segments optionally have the same or similar inner and/or outer diameters.

The first and/or second segments may be elongate, for example, in a direction parallel to a longitudinal axis of the core. The first and second segments may have the same length as each other (length being measured in a direction parallel to the axis of the core), or the first and second segments may be of different lengths from each other. In some embodiments, the first and/or the second segments have a respective length that remains generally uniform over the length of the sleeve. In some embodiments, the first and/or the second segments have a respective length that varies over the length of the sleeve.

At least some of the segments may be affixed to the core to define an integral structure therewith. The segments may be affixed to the core by any suitable technique, for example, adhesive bonding, fusion, or mechanical interference. In some embodiments, all of the segments may be affixed to the core. In some alternative embodiments, at least the segments at, or near, the opposite ends of the sleeve are affixed to the core such that the segments intermediate the fixed segments are retained captive.

In some embodiments, adjacent segments butt each other face to face without mechanical interlocking or keying. In other embodiments, at least some of the adjacent segments include mechanical keying between the confronting ends of adjacent segments. The mechanical keying may, for example, define articulating engagement for enhancing flexure with the core and/or non-rotational engagement capable of transmitting torque from one segment to another.

The invention may be directed to the sub-assembly or structure per se, or to a delivery system comprising the sub-assembly or structure.

Features and advantages of the invention include: (i) ability to provide a flexible structure with flexibility attributed by the smaller elastic modulus; (ii) ability to provide support for transmission of force, especially an axial compression force, therethrough, attributed by the larger elastic modulus; (iii) straightforward and inexpensive assembly; (iv) good reliability required for a medical device insertable into the vascular system; and/or (v) versatility enabling adjustment of the flexibility and/or support characteristics by adjusting the dimensions and/or properties of the segments.

Although certain ideas and features are summarized above and in the appended claims, protection is claimed for any novel feature or idea described herein and/or illustrated in the drawings whether or not emphasis has been placed thereon.

For a better understanding of the present invention, reference is made to the following description, taken in conjunction with the accompanying drawings, in which like reference characters refer to like parts throughout.
Fig. 1 is a schematic illustration of a stent-valve delivery system.
Fig. 2 is a schematic section through a portion of a sub-assembly usable in the stent-valve delivery system.
Fig. 3 is a schematic section similar to Fig.2, showing a first example modification of the segment profiles.
Fig. 4 is a schematic section similar to Figs. 2 and 3, showing a second example modification of the segment profiles.
Fig. 5 is a schematic illustration illustrating a first example of distal portion of the delivery system.
Fig. 6 is a schematic illustration illustrating a second example of distal portion of the delivery system.
Fig. 7 is a schematic illustration illustrating a third example of distal portion of the delivery system.

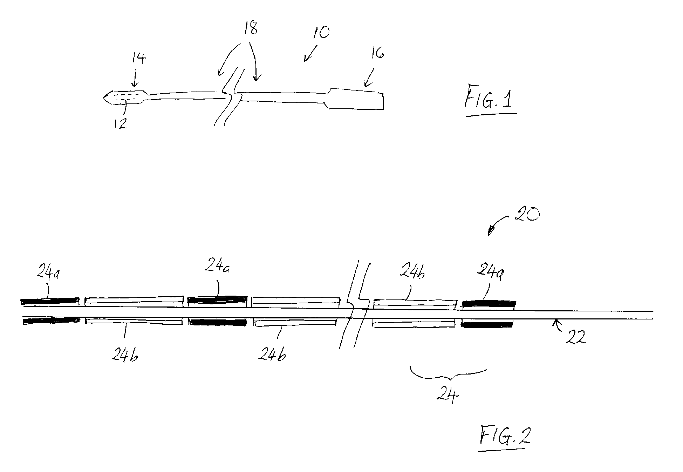

In the following non-limiting detailed description, the same reference numerals are used to denote equivalent or similar features where appropriate. Further, reference is made to the accompanying drawings which form a part hereof, and in which is shown by way of illustration a specific embodiment in which the invention may be practiced. It is to be understood that other embodiments may be utilized and structural changes may be made without departing from the scope of the present invention. Referring to Fig. 1, a delivery catheter 10 is illustrated for introduction into a patient's vasculature for delivering a cardiac stent-valve 12 for implantation to replace an existing cardiac valve. The stent-valve 12 may be delivered in a collapsed condition, and be expandable to a deployed condition at implantation. The stent-valve 12 may be self-expanding, or it may be expandable by application of an expansion force. The stent-valve 12 may, for example, be as described in WO 2009/053497. However, many different types of stent-valve are known in the art, and the invention is not limited to a specific design of stent-valve 12.

The delivery catheter generally comprises a distal portion 14 for introduction into the vasculature, a proximal portion 16 that may remain outside body, and a stem or barrel portion 18 extending between the distal and proximal portions. The distal portion 14 comprises an arrangement for carrying the stent-valve 12 in a collapsed condition for delivery to the implantation site and operable to release or deploy the stent-valve 12 in response to remote manipulation. The proximal portion 16 comprises one or more manually operable controls for manipulating the distal portion 14. The stem portion 18 is configured to be:
(i) flexible to permit tracking of the delivery catheter along the patient's tortuous vasculature (including the extreme bend of the aortic arch), and
(ii) capable of providing mechanical support for:
   (a) enabling the distal portion 14 to be pushed and advanced along the vasculature by pushing force applied to the delivery catheter from outside the body; and
   (b) transmitting a differential, longitudinal displacement force from the proximal portion 16 to the distal portion 14 for manipulating the distal portion 14.

In the prior art, the requirements (i) and (ii) above generally conflict with each other. It has been problematic to find materials for the delivery catheter, especially the stem portion 18, without compromising at least one of the requirements (i) and (ii). For example, if the material is advantageously flexible to satisfy requirement (i), the material may be lack sufficient rigidity for applying longitudinal forces within the stem. This can lead to the material deforming elastically, kinking or buckling, especially where elongate compression and/or elongate pushing force is applied though the material. Alternatively, if the material is relatively stiff to satisfy requirement (ii), this can compromise the ability of the delivery catheter to flex in order to follow a tortuous vasculature, or it may result in undesirable abrasive rubbing against the vassal wall (for example, especially where the catheter passes around the aortic arch, which is often highly stenosed and there is a risk of embolism resulting from calcification rubbed free of the vassal wall and released into the blood stream).

Referring to Fig. 2, some embodiments of the present invention address this issue by providing a structure and/or sub-assembly 20 comprising a flexible support tube 22 and a segmented tube 24 nested one within the other. In the illustrated form, the support tube 22 is arranged as a core, and the segmented tube 24 as a sleeve around the core. However, the relative positions may be swapped if desired. The segments may include plural first segments 24a having a first elastic modulus, and interposed at least one between plural second segments 24b having a second elastic modulus different from the first elastic modulus. In the illustrated form, the segments 24 are arranged in a repeating sequence of a first segment 24a interposed contiguously between second segments 24b adjacent to the fist segment 24a. For example, the segments 24 may define a continuous and/or repeating pattern of alternating first and second segments 24a and 24b, respectively. If desired, additional segments, e.g., third segments (not shown) of a third elastic modulus, may further be included as part of the sequence of segments 24. By way of example, the first elastic modulus may be higher than the second elastic modulus, e.g., by a factor selected from: at least 10 times; at least 100 times; at least 500 times; at least 1000 times.

In Fig. 2, the segments 24 are shown slightly spaced apart from each other, and from the surface of the support tube 22, for ease of visualization. However, it may be appreciated that the segments 24 may directly abut each other and/or may be dimensioned to form a close fit around the support tube 22. The combination of the segments 24 and support tube 22 may define a generally integral structure without substantial play between individual segments and/or between the segments 24 and the support tube 22.

The provision of such segments 24a and 24b provides a structure that can meet better both of the above requirements (i) and (ii). The first segments 24a (of relatively higher elastic modulus) provide stiffness enabling the structure 20 to bear compression loads enabling a longitudinal and/or axial pushing force to be transmitted through the structure, without significant elastic deformation (e.g. compression) even when the structure follows a non-straight path. The second segments 24b (of relatively lower elastic modulus) provide flexibility between the first segments 24a, thereby enabling the structure 20 to retain good flexibility.

In some embodiments, the first segments 24a are of metal, for example, stainless steel. In some embodiments, the second segments 24b are of flexible plastics. The second segments 24b are optionally of the same material as the support tube 22.

The properties of the structure 20 may also be adjusted by the length(s) of the first and second segments 24a and 24b. The segments 24 may be generally elongate in the axial direction of the structure 20. The first and/or second segments 24a and 24b may have a length of less than about 3 cm. The segments 24a and 24b may have the same length or different lengths. In the illustrated form, the first segments 24a (of relatively higher elastic modulus) are shorter than the second segments 24b. For example, the first segments may have a length of about 1cm and/or the second segments 24b may have a length of about 2cm. The length(s) of the first and second segments 24a and 24b may be constant over the length of the structure 20, or the length(s) may vary over the length of the structure 20 to provide different properties over the length.

The segments 24a and 24b may optionally all be affixed to the support tube 22 to define an integrated structure. Alternatively, respective segments 24a and 24b may be affixed in certain affixed zones of the structure 20 leaving other segments captive between the affixed zones. For example, the affixed zones may be or include regions at the opposite ends of the structure 20.

The structure 20 may be assembled easily and cost effectively by sliding the segments 24a and 24b onto the support tube 22, so that the structure does not add significantly to the cost of the delivery device.

The structure 20 may define a tubular lumen 26 therewithin, for passage therethough of one or more other members. In some embodiments, the tubular lumen 26 is a guide-wire receiving lumen for passage of a guide wire through the delivery system 10.

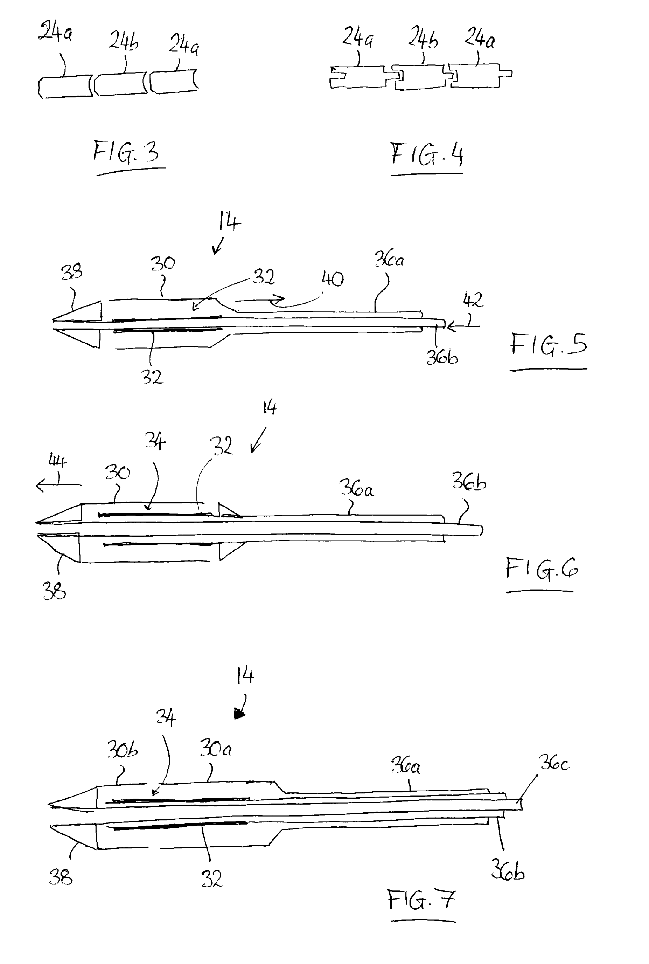

In the form illustrated in Fig. 2, the confronting ends of adjacent segments 24 may be generally flat, such that ends abut each other face to face without any mechanical keying. Referring to Figs. 3 and 4, in some embodiments, the ends may include mechanical keying. For example, in Fig. 3, the end of one segment 24 may fit partly within a recess or pocket of an adjacent segment 24. The end of one segment may be beveled or rounded. Such an arrangement may optionally provide support for transmitting longitudinal compression force, while also facilitating bending articulation. Referring to Fig. 4, the mechanical keying may additionally or alternatively include non-rotation keying for transmitting torque from one segment between adjacent segments 24.

Figs. 5-7 illustrate examples of different distal portion 14 of the delivery device 10, to show how the structure 20 may advantageously be used. The distal portion 14 may optionally comprise at least one containment sheath part 30 and/or a stent-holder (indicated schematically at 32). The distal portion may define a stent containing region 34. The sheath part 30 may be configured for containing a stent-valve 12 (from Fig. 1) in a collapsed condition. For example, the sheath part(s) 30 may encompass at least a portion of the stent-valve 12 to contain the stent-valve and/or constrain the stent-valve 12 against expansion. The stent-holder 32 may comprise one or more elements, stops and/or surfaces configured to restrain the stent-valve 12 against axial movement in at least one (and preferably both) axial direction(s). The distal portion 14 may be configured to release the stent-valve 12 from the stent containing region 34 by displacement of the sheath part(s) 30 relative to the stent-holder 32. The displacement is driven by movement of respective control members at the proximal portion 16, which movement is transmitted through the stem 18 to the distal portion 14. The stem portion 18 comprises plural tubular members 36 nested one within another, and relatively displaceable to transmit displacement forces from the proximal portion 16 to the distal portion 14. One or more of the tubular members 36 may comprise the structure 20 described above, which is especially useful as a tubular member for transmitting a longitudinal pushing, or compressive force to the distal portion 14.

In the example of Fig. 5, the distal portion comprises a single sheath part 30 that is displaceable in a proximal direction (indicated by arrow 40) relative to the stent holder 32 to open the stent containing region 34. A distal tip 38 may be fixed relative to the stent holder 32. The tubular members 36 include an (e.g. outer) tubular member 36a coupled to the sheath part 30. The structure 20 may be used as an (e.g. inner) tubular member 36b for restraining the stent holder 32 by application of a longitudinal pushing force (indicated by arrow 42) when the sheath part 30 is pulled back by the outer tubular member 36a.

In the example of Fig. 6, the distal portion comprises a single sheath part 30 that is displaceable in a distal direction (indicated by arrow 44) relative to the stent holder 32 to open the stent containing region 34. The distal tip 38 may be fixed relative to the sheath part 30. The tubular members may comprise an (e.g. outer) tubular member 36a for restraining the stent holder 32. The structure 20 may be used as an (e.g. inner) tubular member 36b coupled to the sheath part 30 to apply the pushing force to the sheath part 30.

In the example of Fig. 7, the distal portion comprises first and second sheath parts 30a and 30b that move in opposite directions to open the stent containing region 34. The first sheath part 30a is similar to the sheath illustrated in Fig 5. The second sheath part 30b is similar to the sheath illustrated in Fig. 6. The tubular members 36 include an (e.g. outer) tubular member 36a coupled to the first sheath part 32a, an (e.g. inner) tubular member 36b coupled to the second sheath part 32b, and an (e.g. central tubular member 36c coupled to the stent holder 32. The inner tubular member 36b may optionally comprise a respective structure 20 described above useful for transmitting a pushing force for advancing the second sheath part 30b when opening the second sheath part 30b. Additionally or alternatively, the central tubular member 36c may optionally comprise a respective structure 20 useful for transmitting a restraining (pushing) force to the stent holder 32 when opening the first sheath part 30a. The first and second sheath parts 30a and 30b may be configured for displacement individually in separate sequence, for example, for sequential release of the stent-valve.

Although the above description illustrates a delivery catheter for delivery a stent-valve, it will be appreciated that the same principles may be used in a flexible delivery catheter for delivering other types of stents. In particular, the delivery catheter may be suitable for stent grafts, for example, for treating a thoracic aortic aneurism and/or an abdominal aortic aneurism.

It is emphasized that the foregoing description is merely illustrative of preferred embodiments that do not limit the scope of the invention. Many modifications, equivalents and improvements may be used within the scope and/or principles of the invention.

## Claims

1. A sub-assembly for use within a stent delivery catheter, the sub-assembly for transmitting an axial pushing force within the stent delivery catheter, the sub-assembly comprising a flexible support tube and a segmented tube nested one within the other, the segmented tube comprising segments arranged contiguously end to end, the segments comprising plural first segments having a first elastic modulus interposed at least one between plural second segments having a second elastic modulus different from the first elastic modulus.

2. The sub-assembly of claim 1, wherein the support tube is nested with the segmented tube.

3. The sub-assembly of claim 1 o 2, wherein the segments are arranged in repeating sequence of a first segment interposed contiguously between second segments adjacent to the first segment.

4. The sub-assembly of claim 1, 2 or 3, wherein the segments define a continuous sequence of alternating first and second segments.

5. The sub-assembly of claim 1, 2, 3 or 4, wherein the first elastic modulus is greater than the second elastic modulus by a factor selected from: at least 10 tens; at least 100 times; at least 500 times; at least 1000 times.

6. The sub-assembly of any preceding claim, wherein the first segments are of metal, and the second segments are of plastics.

7. The sub-assembly of any preceding claim, wherein the second segments are of the same material as the support tube.

8. The sub-assembly of any preceding claim having an axial length of at least 1m.

9. The sub-assembly of any preceding claim, wherein the sub-assembly is flexible.

10. A stent delivery catheter comprising a sub-assembly as defined in any preceding claim.

11. The stent delivery catheter of claim 10, wherein the delivery catheter is a stent-valve delivery catheter.

12. The stent delivery catheter of claim 10 or 11, wherein the delivery catheter includes a proximal portion and a distal portion, the distal portion comprising a sheath part that, in use, is advanced by pushing the sheath in a distal direction for opening a stent containing region of the distal portion, and wherein the sub-assembly is coupled to the sheath part for transmitting a pushing force from the proximal portion to the distal portion.
